# EUROPEAN PATENT APPLICATION

(11) **EP 1 738 760 A1**
(43) Date of publication of application: **03.01.2007**
(21) Application number: 06253370.8
(22) Date of filing: 28.06.2006
(51) Int. Cl.: A61K 31/77, A61P 29/00

(54) **Use of biocompatible amphiphilic polymers as an anti-inflammatory agent**

(30) Priority: 29.06.2005 US 170411
(71) Applicant: LifeScan, Inc., Milpitas, CA 95035 (US)
(72) Inventor: Fung, Ramie, Flemington, NJ 08822 (US)
(74) Representative: Tunstall, Christopher Stephen

(57) **Abstract**

The present invention relates to the use of bioabsorbable and biocompatible amphiphilic polymers as an anti-inflammatory agent. In particular, the present invention provides methods of treating inflammatory diseases or conditions by employing an amphiphilic polymer made of a polybasic acid or a derivative thereof, a monoglyceride and a polyether.

## Description

### FIELD OF THE INVENTION

The present invention relates to the use of bioabsorbable and biocompatible amphiphilic polymers as an anti-inflammatory agent. In particular, the present invention provides methods of treating inflammatory diseases and methods of preventing or reducing inflammation that occurs in tissue transplantation.

### BACKGROUND OF THE INVENTION

Three major events occur at the start of the inflammatory response: blood supply to the area is increased; capillary permeability is increased allowing soluble mediators of immunity to reach the site of inflammation; and leukocytes migrate out of the venules into the surrounding tissues.

Neutrophils are generally the first cells to reach the site of inflammation as part of the acute inflammation response. The neutrophils represent the majority of the blood leukocytes and are responsible for phagocytosis. They release inflammatory cytokines such as Tumor Necrosis Factor α ("TNFα") and Interleukin 1("IL-1"), both of which attract more neutrophils as well as other leukocytes. Among these are the T cells.

T cells have a wide range of activities, including the control of B lymphocyte development and antibody production, interaction with antigen presenting cells, and even phagocytosis. A subset of T cells, T Helper (T_{H}) cells, interact with antigen-presenting cells, such as neutrophils, and cytokines, such as those described above, are released.

Cytokine is a term which encompasses a large group of molecules involved in signalling between cells, and include interferons, interleukins, colony-stimulating factors, and chemokines. Cytokines specifically released by interaction with the T_{H} Cells include Interferon γ (IFNγ), TNFβ, IL-2, IL-3 and TNFα. With the release of these pro-inflammatory cytokines, the inflammatory response is continually perpetuated.

In the treatment of inflammatory diseases, these cytokines are targeted and their production is dampened down in order to relieve the symptoms associated with those diseases. For example, Remicade© (infliximab, Centocor, Inc.) is a monoclonal antibody that specifically binds to soluble and transmembrane forms of human TNFα and inhibits the ability of TNFα to bind to its receptors. This inhibition prevents the induction of the inflammatory response and is currently being used to treat Rheumatoid Arthritis and Crohn's Disease. As another example, aspirin has been used as an inhibitor of cyclooxygenease 2 (COX-2) to dampen inflammatory responses mediated by COX-2.

Troglitazone, a member of a class of compounds known as thiazolidinediones (TZDs) currently being used as an antidiabetic agent, is being evaluated for its potential as an anti-inflammatory agent. Troglitazone binds to peroxisome proliferator-activated receptor gamma (PPARγ, a nuclear receptor), resulting in the formation of a heterodimer of PPARγ and the retinoid x receptor (RXR). The heterodimer mediates a variety of cellular effects including regulation of adipocyte differentiation, lipid metabolism and glucose homeostasis, thereby increasing insulin sensitivity in Type 2 Diabetic patients. PPARγ is expressed on adipose tissue, skeletal muscle, adrenal gland, colonic epithelium, heart, pancreas, liver, vascular smooth muscle cells and macrophages. Acting as PPARγ agonists, the TZDs have been shown to inhibit the production of inflammatory cytokines. Other PPARγ agonists include 15 deoxy-Δ^{12,14} prostaglandin J₂ and oxidized phospholipids/lipoproteins.

The use of PPARγ agonists to inhibit or decrease inflammatory responses has been described in the literature. PPARγ agonists have been shown to inhibit the production of IL-4 in CD4⁺ T cells (Chung et at., Mol. Pharmacol. 64(5): 1169-79, 2003), the production of TNF-α and IL-1β in THP-1 cells (a monocyte cell line) and in human rheumatoid arthritis synoviocytes (Ji et al., J. Autoimmun. 17(3): 215-21, 2001). PPARγ agonists have also been shown to inhibit the production of IL-2 and IFNγ (Clark et al., J. Immunol. 164(3): 1364-71, 2000; Cunard et al., J. Immunol.168: 2795-2802, 2002). Jiang et al. (Nature 391: 79-82, 1998) has also reported inhibition of IL-1β, IL-6 and TNF-α production by PPARγ agonists. In addition to the inhibition of cytokine production, PPARγ agonists have also been shown to decrease the production of other molecules involved in an inflammatory response, for example, COX-2, prostaglandin E₂, and inducible nitric oxide synthase (Mendez et al., Hypertension.42 (4): 844-50, 2003; Cuzzocrea et al., Eur. J. Pharmacol. 483 (1): 79-93, 2004).

Yu et al. (Biochim Biophys Acta. 1581(3): 89-99, 2002) reported that conjugated linoleic acid was able to decrease the production of pro-inflammatory products in macrophages in a manner similar to the actions of PPARγ agonists. Krey et al. suggested using fatty acids as PPARγ agonists (Mol. Endocrinol. 11(6): 779-91, 1997).

Bioabsorbable and biocompatible polymers have been used in the manufacture of devices for use as drug delivery vehicles, cellular delivery vehicles, growth constructs, wound closures, sutures, and adhesion prevention barriers, among others. See, e.g., U.S. Published Application 2001/0053778A1, U.S. Patent 6,521,736 B2, EP 1 369 136 A1, EP 1 270 024 A1, and EP 1 348 451 A1. It has not been recognized, however, that these polymers have any inhibitory effect on cytokine production or on inflammatory reactions. In fact, the introduction of some polymers into the body and/or the breakdown by hydrolysis has induced cytokine synthesis, causing an inflammatory response to occur. See, for example, Taira et al. (J. Oral Rehabil. 30(1):106-9, 2003); Sung et al., Biomaterials 25(26):5735-42, 2004; Fournier et al., Biomaterials 24(19):3311-31, 2003).

U.S. Patent 6,689,350 B2 describes the formation of polyesters and polyamides, wherein the backbones of the polymers have incorporated certain biologically active compounds. The biologically active compounds are released upon hydrolysis of the polymers. U.S. Patent 6,685,928 B2 describes the use of aromatic polyanhydrides in medical devices, which, upon degradation, produce certain bioactive agents such as salicylic acid.

### SUMMARY OF THE INVENTION

The present invention provides a method for treating an inflammatory condition in a subject by administering an amphiphilic polymer made of a polybasic acid or a derivative thereof, a monoglyceride and a polyether.

Inflammatory conditions which can be treated in accordance with the present invention includes various forms of arthritis and other degenerative bone and joint diseases and injuries; inflammatory immune disorders such as rheumatic diseases, allergic disorders, asthma, acute respiratory distress syndrome, chronic obstructive pulmonary disease, allergic rhinitis, skin disorders, multiple sclerosis, gastrointestinal disorders such as Crohn's disease and ulcerative colitis, poststreptococcal and autiommune renal failure, septic shock, systemic inflammatory response syndrome (SIRS), and envenomation. Additional diseases that can be treated include diabetes, hyperlipidemia, coronary heart disease, cancer or proliferative disease. The present methods can also be used to reduce or prevent systemic or local inflammation in such diseases as vascular diseases, atherosclerosis, systemic lupus erythematosis (SLE), acute respiratory distress syndrome; and reduce or prevent swelling occurring after injury and surgical procedures.

The present methods are particularly useful for preventing or inhibiting inflammatory responses during or after tissue or cell transplantation, for example, islet transplantation, thereby improving cell survival and growth after transplantation.

Preferred polymers for use in the present methods are those capable of inhibiting the production of pro-inflammatory cytokines, such as IL-1, IL-6, IL-12 and TNF-alpha. Especially preferred polymers are those that inhibit the production of pro-inflammatory cytokines by acting as a PPARγ agonist.

Depending upon the disorder or condition to be treated, the polymers can be prepared and processed into various forms suitable for administration to the subject, such as injectable microdispersions, medical or surgical devices in forms such as filaments, films or molded articles, or as coatings of such devices.

In certain specific embodiments of the present invention, the polymer is used in conjunction with one or more other bioactive agents, where the polymer functions both as an anti-inflammatory agent and as a delivery vehicle for the bioactive agents. Bioactive agents contemplated for use in conjunction with the polymer include tissues or cells prepared for implantation, and other anti-inflammatory compounds, useful for treating diseases or disorders associated with inflammation.

In a specific embodiment, a matrix or scaffold is prepared using the polymer, which carries cells, preferably islets or cells engineered to produce insulin, to a suitable transplantation site.

In still another embodiment, a polyether alkyd polymer is used to coat another biodegradable, bioabsorable matrix or scaffold, and provide the matrix or scaffold with an independent anti-inflammatory property.

A polymer can be administered to the subject via an appropriate route, depending upon the specific condition being treated and the dosage form of the polymer.

### BRIEF DESCRIPTION OF THE DRAWINGS

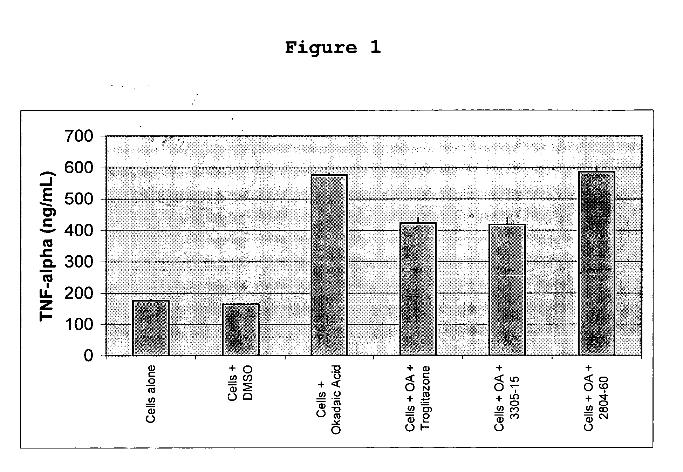

**Figure 1** illustrates inhibition of TNF-α release from monocytes by troglitazone and MGSA polymer.

### DETAILED DESCRIPTION OF THE INVENTION

The present invention is based on the unique recognition that bioabsorbable and biocompatible amphiphilic polymers, particularly polymers made of a polybasic acid or a derivative thereof, a monoglyceride and a polyether, inhibit the production of pro-inflammatory mediators and therefore inhibit inflammatory responses. Accordingly, the present invention provides therapeutic methods which employ amphiphilic polymers for treating inflammatory disorders, and for preventing or inhibiting inflammatory responses. The methods of the present invention are particularly useful for preventing or inhibiting inflammatory responses associated with tissue or cell transplantation, for example, islet transplantation.

Various bioabsorbable and biocompatible polymers have been described for use in the manufacture of drug or cell delivery devices or growth constructs. However, inflammatory responses are expected to occur with the introduction of a foreign object into the body. In fact, some polymers have been reported to induce cytokine synthesis, causing an inflammatory response to occur.

The present inventor has unexpectedly identified that bioabsorbable and biocompatible amphiphilic polymers made of a polybasic acid or a derivative thereof, a monoglyceride and a polyether, have an anti-inflammatory effect and are therefore useful for treating disorders and conditions associated with undesirable inflammatory responses.

Without intending to be bound by any particular theory, it is believed that the anti-inflammatory effect of the amphiphilic polymers is attributable to the fatty acid component of the monoglyceride. Additionally, it is believed that the anti-inflammatory effect of the amphiphilic polymers is mediated through PPARγ, i.e., the polymers or their hydrolysis products act as PPARγ agonists to inhibit the production of pro-inflammatory mediators, especially pro-inflammatory cytokines.

As used herein, the term "cytokine" refer to proteins made by cells that affect the behavior of other cells; for example, interferons, interleukins, colony-stimulating factors, and chemokines. Pro-inflammatory cytokines, i.e., cytokines that mediate or participate in an inflammatory response, include IFNγ, IL-1, IL-2, IL-3, IL-6, TNFα, TNFβ, among others.

According to the present invention, biocompatible and bioabsordable amphiphilic polymers having an anti-inflammatory effect are the reaction product of a polybasic acid or derivative thereof, a monoglyceride and a polyether. Such polymers may be classified as polyether alkyds. The compositions and manufacture of these polymers have been described in EP 1 369 136 A1, which is incorporated herein by reference. Such polymers have an aliphatic polyether/polyester backbone with pendant fatty acid ester groups. The ester linkages in the alkyd block are hydrolytically unstable, when the polymer is exposed to moist body tissue it readily breaks down into small segments. Hydrolysis of these polymers is expected to give rise to glycerol, a water-soluble polyether, dicarboxylic acid(s) and fatty acid(s), all of which are biocompatible. In this regard, while co-reactants could be incorporated into the reaction mixture of the polybasic acid and the diol for the formation of the polyether alkyds, it is preferable that the reaction mixture does not contain a concentration of any co-reactant, which would render the subsequently prepared polymer nonabsorbable.

Monoglycerides that can be used to prepare the polymers suitable for use in the present invention include, without limitation, monostearoyl glycerol, monopalmitoyl glycerol, monomyrisitoyl glycerol, monocaproyl glycerol, monodecanoyl glycerol, monolauroyl glycerol, monolinoleoyl glycerol, monooleoyl glycerol, and combinations thereof. Preferred monoglycerides include monooleoyl glycerol, monopalmitoleoyl glycerol and monolinoleoyl glycerol. Use of monoglycerides with long-chain saturated fatty acids results in polymers that are polymeric waxes, i.e., solid substances having a low-melting point of between 25°C and 70°C. Alternatively, use of monoglycerides with short-chain or unsaturated fatty acids results in liquid polymers, i.e., polymers that are liquid at room temperature and have a melting point of about 25°C or lower.

Polybasic acids that can be used to prepare the polymers suitable for use in the present invention include natural multifunctional carboxylic acids, such as succinic, glutaric, adipic, pimelic, suberic, and sebacic acids; hydroxy acids, such as diglycolic, malic, tartaric and citric acids; and unsaturated acids, such as fumaric and maleic acids. Polybasic acid derivatives include anhydrides, such as succinic anhydride, diglycolic anhydride, glutaric anhydride and maleic anhydride, mixed anhydrides, esters, activated esters and acid halides.

Polyethers that can be used to prepare the polymers suitable for use in the present invention include any commonly used water-soluble linear or branched alkylene oxide known in the art and is preferably a poly(ethylene oxide), poly(propylene oxide) or poly(tetra methylene oxide). Poly(alkylene oxide) blocks containing ethylene oxide, propylene oxide or tetramethylene oxide units in various combinations can also be used to prepare the polymers.

Polymerization of the polyether alkyds preferably is performed under melt polycondensation conditions in the presence of an organometallic catalyst at elevated temperatures. The organometallic catalyst preferably is a tin-based catalyst, e.g., stannous octoate. Preferably, the catalyst is present in the mixture at a mole ratio of polyol and polycarboxylic acid to catalyst in the range of from about 15, 000/1 to 80,000/1. The reaction is typically performed at a temperature not lower than about 120°C. Higher polymerization temperatures give rise to polyether alkyds with higher molecular weights, which are desirable for numerous applications. Those skilled in the art can choose specific reaction conditions such as temperature, time and pressure, taking into consideration a number of factors including, e.g., the chemical and mechanical properties of the polyether alkyd polymers desired, the viscosity of the reaction mixture, and the melting temperature of the polymer substrates. Generally, the reaction mixture is maintained at about 180°C. The polymerization reaction can be allowed to proceed at this temperature until the desired molecular weight and percent conversion is achieved for the polyether alkyd product, which can take from about 15 minutes to 24 hours. Increasing the reaction temperature generally decreases the reaction time needed to achieve a particular molecular weight.

Multifunctional monomers can be used to produce crosslinked polymeric networks. Polymers having pendant hydroxyls can be synthesized using a hydroxy acid such as malic or tartaric acid in the synthesis. Polymers with pendent amines, carboxyls or other functional groups also can be synthesized. Alternatively, double bonds can be introduced by using monoglycerides or diacids containing at least one double bond to allow photocrosslinking. Hydrogels can be prepared using this approach provided the polymer is sufficiently water soluble or swellable. A variety of biological active compounds can be covalently attached to these functional polymers by known coupling chemistry to give sustained release of the bioactive compounds.

Endcapping reactions can impart new functionality to the polymers. Endcapping reactions convert the terminal and pendant hydroxyl groups and terminal carboxyl groups into other types of chemical moieties. Typical endcapping reactions include, but are not limited to, alkylation and acylation reactions using common reagents such as alkyl, alkenyl, or alkynyl halides and sulfonates, acid chlorides, anhydrides, mixed anhydrides, alkyl and aryl isocyanates and alkyl and aryl isothiocyanates. For instance, when acryloyl or methacryloyl chloride is used to endcap these polymers, acrylate or methacrylate ester groups, respectively, are created that subsequently can be polymerized to form a crosslinked network.

Copolymers can be prepared by using mixtures of diaacids, different monoalkanoyl glyceriders and different polyethers to obtain desired properties. Similarly, two or more polyether alkyds can be prepared to tailor properties for different applications.

Those skilled in the art, having the benefit of the disclosure herein, can ascertain particular properties of the polymers required for particular purposes and readily prepare polymers that provide such properties.

The polymers, once prepared, can be tested by various *in vitro* tests to confirm their anti-inflammatory activities. For example, a polymer can be added to cultured cells capable of releasing one or more pro-inflammatory cytokines, and the effect of the polymer on the release of the cytokines can be readily determined. The polymer can also be tested in an adipogenicity assay to determine its ability to potentiate the activity of PPARγ.

In accordance with the present invention, preferred polymers are those that inhibit the production of pro-inflammatory cytokines, in particular, IL-1, IL-6, IL-12 and TNF-alpha. Especially preferred polymers are those that inhibit the production of pro-inflammatory cytokines by acting as a PPARγ agonist.

Based on the unique recognition of the anti-inflammatory effect of the amphiphilic polyether alkyd polymers, the present invention provides methods for treating inflammatory disorders or inflammatory conditions in a subject by employing the amphiphilic polyether alkyd polymers.

By "subject" is meant to include any mammalian subjects, particularly human subjects.

By "treating" is meant reducing, inhibiting, preventing, or slowing down the development or progression of systemic or local inflammation, thereby ameliorating the symptoms or severity of a disorder.

Disorders that can be treated by employing the polymers described above include arthritis and other degenerative bone and joint diseases and injuries, including but not limited to rheumatoid arthritis, osteoarthritis, ankylosing spondylitis, psoriasis, psoriatic arthritis, systemic lupus erythematosus, juvenile arthritis; tendonitis, ligamentitis and traumatic joint injury. Additional disorders that can be treated by employing the polymers described above include inflammatory immune disorders, including but not limited to rheumatic diseases, allergic disorders, asthma, acute respiratory distress syndrome, chronic obstructive pulmonary disease, allergic rhinitis, skin disorders, multiple sclerosis, gastrointestinal disorders such as Crohn's disease and ulcerative colitis, poststreptococcal and autiommune renal failure, septic shock, systemic inflammatory response syndrome (SIRS), and envenomation. Additional diseases that can be treated include diabetes, hyperlipidemia, coronary heart disease, cancer or proliferative disease.

The amphiphilic polyether alkyds can also be employed to reduce or prevent systemic or local inflammation in such diseases as vascular diseases, atherosclerosis, systemic lupus erythematosis (SLE), acute respiratory distress syndrome; reduce or prevent swelling occurring after injury and surgical procedures.

According to the present invention, the amphiphilic polyether alkyds are particularly useful for preventing or inhibiting inflammatory responses associated with tissue or cell transplantation, for example, islet transplantation, thereby improving cell survival. Implanted islets can be exposed to an early, nonspecific inflammatory injury triggered by activation of the transplant microenvironment. Resident islet macrophages may also release pro-inflammatory cytokines that are detrimental to islet cell survival and function. Thus, the amphiphilic polyether alkyds can inhibit the production of pro-inflammatory cytokines and improve the survival and function of transplanted islet cells.

Depending upon the disorder or condition to be treated, polyether alkyds can be provided in various forms for administration to the subject.

In one embodiment, the polyether alkyds are provided as injectable microdispersions, including microemulsions or micelles, formed from liquid polymers or polymeric wax.

In another embodiment, the polymers are melt-processed and provided in the form of a device to a subject.

For example, the polymers can be injection or compression molded to make implantable medical and surgical devices, especially wound closure devices, such as surgical clips, staples and sutures.

Alternatively, the polyether alkyds can be extruded to prepare filaments. The filaments thus produced can be fabricated into sutures or ligatures, attached to surgical needles, packaged, and sterilized by known techniques. The polymers of the present invention can be spun as monofilament or multifilament yarn and woven or knitted to form sponges or gauze, or used in conjunction with other molded compressive structures as prosthetic devices within the body of a mammal such as human where it is desirable that the structure have high tensile strength and desirable levels of compliance and/or ductility. Non-woven sheets also can be prepared and used as described above. Useful embodiments include tubes, including branched tubes, for artery, vein or intestinal repair, nerve splicing, tendon splicing, sheets for taping-up and supporting damaged surface abrasions, particularly major abrasions, or areas where the skin and underlying tissues are damaged or surgically removed.

Additionally, the polymers can be molded to form films which, when sterilized, are useful as adhesion prevention barriers. Another alternative processing technique for the polymers of this invention includes solvent casting, particularly for those applications where a drug delivery matrix is desired.

In accordance with the present invention, the surgical and medical uses of the filaments, films, and molded articles of the polymers include, but are not limited to, knitted products, woven or non-woven, and molded products including, but not limited to, burn dressings, hernia patches, meshes, medicated dressings, fascial substitutes, gauze, fabric, sheet, felt or sponge for liver hemostasis, gauze bandages, arterial graft or substitutes, bandages for skin surfaces, suture knot clip, orthopedic pins, clamps, screws, plates, clips, e.g. for vena cava, staples, hooks, buttons, snaps, bone substitutes, e.g. as mandible prosthesis, intrauterine devices, e.g. as spermicidal devices, draining or testing tubes or capillaries, surgical instruments, vascular implants or supports, e.g., stents or grafts, or combinations thereof, vertebral discs, extracorporeal tubing for kidney and heart-lung machines, artificial skin, and supports for cells in tissue engineering applications.

In another embodiment, the polyether alkyd polymers are used to coat a surface of a medical device to provide an independent anti-inflammatory effect or enhance the anti-inflammatory effect of the medical device or the bioactive agent contained therein. The polymer may be applied as a coating using conventional techniques. For example, the polymer may be solubilized in a dilute solution of a volatile organic solvent, such as acetone, methanol, ethyl acetate or toluene, and then the article can be immersed in the solution to coat its surface. Once the surface is coated, the surgical article can be removed from the solution where it can be dried at an elevated temperature until the solvent and any residual reactants are removed.

Although the amphiphilic polymers possess independent anti-inflammatory activities, the polymers can be used in conjunction with other bioactive agents to achieve more effective treatment.

By "other bioactive agents" is meant compounds or materials having a biological activity separate from the amphiphilic polymers, including tissues or cells for implantation and other anti-inflammatory compounds for treating diseases or disorders associated with inflammation. Anti-inflammatory compounds suitable for use in conjunction with the amphiphilic polymers of the present invention include COX-2 inhibitors, tetracycline compounds, non-steroidal anti-inflammatory drugs (NSAID), corticosteroids, matrix metalloprotease inhibitors, local anaesthetics, glucosamine, chondroitin sulfate and collagen hydrolysate. See, e.g., U.S. Patent 6,677,321 and 6,245,797. Where an amphiphilic polymer is used in conjunction with one or more other bioactive agents, the amphiphilic polymer can function both as an anti-inflammatory agent and as a delivery vehicle. For example, microemulsions or micelles prepared from a liquid polymer or polymeric wax are desirable for delivery of poorly soluble bioactive agents that have poor bioavailability. Polymers prepared in the form of filaments, films, and molded articles can serve as a substrate to facilitate the application of other bioactive compounds to treat injury or swelling.

In a specific embodiment, the polymers and blends thereof are used in tissue engineering applications. In this embodiment, the polymers not only provide the desired anti-inflammatory effect to reduce or prevent inflammatory responses, but also serve as vehicle or scaffold for cell delivery and growth.

It should be appreciated that the recognition of the independent anti-inflammatory activities of the polyether alkyd polymers permits a selective use of these polymers as a vehicle for delivery of cells in connection with treatment of inflammatory disorders or conditions associated with inflammatory responses that result in poor survival of the cells. In particular, the polymers of the present invention could be used to prevent tissue rejection that occurs with transplantation, such as islet transplantation.

In a preferred embodiment, a polyether alkyd polymer is used in the preparation of a matrix or scaffold, which is used to carry cells, preferably islets or cells engineered to produce insulin, to a suitable transplantation site.

In another preferred embodiment, a polyether alkyd polymer is used to coat another biodegradable, bioabsorable matrix or scaffold, and provide the matrix or scaffold with an independent anti-inflammatory property.

Appropriate tissue scaffolding structures and their manufacture are known in the art. See, for example, U.S. Pat. No. 5,306,311, which describes prosthetic articular cartilage; WO 94/25079, which describes porous biodegradable scaffolding; WO 93/08850, which describes prevascularized implants; all of which are hereby incorporated by reference.

Methods of seeding and/or culturing cells in tissue scaffoldings are also known in the art. See, e.g., EP 422 209 B1, WO 88/03785, WO 90/12604 and WO 95/33821, all of which are hereby incorporated by reference.

The polymers, whether provided alone or in conjunction with one or more other bioactive agents, can be combined with a pharmaceutically acceptable carrier. A pharmaceutically acceptable carrier means any of the standard carriers, including any and all solvents, dispersion media, coatings, bandages, patches, antibacterial and antifungal agents, isotonic, absorption delaying or enhancing agents, transport polypeptides and lipids, and the like. Except insofar as any conventional media or agent is incompatible with the active ingredient, its use is contemplated.

The polymers can be administered to a subject via any standard route, taking into consideration the disorder or condition to be treated and the dosage form in which the polymer is provided. Generally speaking, the polymer can be administered via oral, parenteral, pulmonary, buccal, nasal, ocular, topical, and vaginal routes, or as a suppository. Bioerodible particles, ointments, gels, creams, and similar soft dosage forms adapted for the administration via the above routes may also be formulated. Other modes of administration, e.g. transdermal, and compositional forms, e.g. more rigid transdermal forms, are within the scope of the invention as well. For delivery to specific sites of inflammation, other means can be used for administering the composition such as, for example, by intraarticular, periarticular or intraosseous injection. Where tissue engineering or transplantation is involved, the polymers or a device containing the polymers can be implanted or injected to a selected site.

The present invention is further illustrated without limitation by the following examples.

### EXAMPLE 1

### MGSA Polymer Inhibited Cytokine Release From Monocytes

Human monocytes were isolated from freshly collected buffy coat preparations of whole human blood. Briefly, whole blood was diluted 1:1.33 with sterile phosphate buffered solution (PBS) and layered over Histopaque, which was then centrifuged for 30 minutes at 1500 rpm at room temperature. The interface was harvested and washed with PBS, followed by another centrifugation at 1500 rpm for 10 minutes. A final wash was performed in RPMI 1640 with 10% fetal bovine serum (FBS). Cells were counted using a hemacytometer and seeded at 2.4x10⁶ cells/ 300 µl into a 48-well tissue culture treated plate. Cells were incubated for 1 hour at 37°C, 5% CO₂ and non-adherent cells were removed with a PBS wash. 300µl of RPMI 1640 with 10% FBS was added back to the cells along with the various test conditions; cells in media alone, with DMSO (the vehicle used to reconstitue okadaic acid and troglitazone), with okadaic acid (OA, 50 nM), with OA and troglitazone (30 µm), with OA and monostearyl glyceride copolymerized with succinic anhydride (MGSA)-liquid (3305-15, 50%), and with OA and MGSA-solid (2804-60, 50%). The supernatants were collected after overnight incubation at 37°C, 5% CO₂ and later analyzed for TNF-α by ELISA.

The results shown in **Table 1** and **Figure 1** demonstrate that troglitazone significantly decreased (P<0.01) the amount of TNF-α released by monocytes in the presence of OA and that the MGSA-liquid (3305-15) had the same effect as troglitazone. MGSA-solid (2804-60) showed no effect in the inhibition of cytokine production.

**Table 1**

| | TNF-α (ng/ml) | Std. Dev. |
|---|---|---|
| Cells alone | 176.652 | 2.627 |
| Cells + DMSO | 165.256 | 0.0832 |
| Cells + Okadaic Acid | 575.765 | 5.657 |
| Cells + OA + Troglitazone | 422.554 | 16.3 |
| Cells + OA + 3305-15 | 418.272 | 20.664 |
| Cells + OA + 2804-60 | 585.957 | 18.064 |

### Example 2

### Use Of MGSA Polymer To Promote Adipogenesis

PPARγ plays a major role in adipogenesis. In order to determine whether MGSA polymer could act as a PPARγ agonist, it was evalutated in an adipogenicity assay.

3T3-L1 cells (ATCC, CL-173) were grown to confluency in culture medium consisting of Dulbecco's Modified Eagle's Medium (DMEM) supplemented with 10% fetal bovine serum, 100 units/ml penicillin, and 100µg/ml streptomycin. Two days post confluency (day 0), 1.7µM insulin, 0.5µM dexamethasone, and 0.5mM isobutylmethylxanthine (collectively designated IDX) were added to the culture medium (differentiation medium). Following two days of culture at 37°C, 5% CO₂, the medium was changed to culture medium supplemented with 1.7µM insulin alone (maintenance medium). On day 7, the cells were fixed with 10% formalin and stained for lipid accumulation using Oil Red O. The control group was differentiated using the protocol described above. The test groups followed the same protocol but used either synthetic (thiazolidinediones) or natural (15 deoxy Δ^{12,14} prostaglandin J₂) PPARγ agonists at 5µM, or MGSA in the differentiation medium instead of IDX with no media change into maintenance medium.

## Claims

1. The use an amphiphilic polymer for the manufacture of a medicament for treating an inflammatory condition, wherein said polymer is the reaction product of a polybasic acid or a derivative thereof, a monoglyceride, and a polyether.

2. A method for treating an inflammatory condition in a subject comprising administering to said subject an amphiphilic polymer, wherein said polymer is the reaction product of a polybasic acid or a derivative thereof, a monoglyceride, and a polyether.

3. The use of claim 1 or method of claim 2, wherein said inflammatory condition is selected from the group consisting of rheumatoid arthritis, osteoarthritis, ankylosing spondylitis, psoriasis, psoriatic arthritis, asthma, acute respiratory distress syndrome, chronic obstructive pulmonary disease, and multiple sclerosis.

4. The use of claim 1 or method of claim 2, wherein said inflammatory condition is associated with a vascular disease, atherosclerosis, systemic lupus erythematosis (SLE), acute respiratory distress syndrome, or an injury, or has occurred during or after a surgical procedure.

5. The use of claim 1 or method of claim 2, wherein said inflammatory condition has occurred during or after tissue or cell transplantation.

6. The use of claim 1 or method of claim 2, wherein said polymer inhibits the production of a pro-inflammatory cytokine selected from the group consisting of IL-1, IL-6, IL-12 and TNF-alpha.

7. The use of claim 1 or method of claim 2, wherein said polymer is a liquid or wax polymer, and is prepared as an injectable microdispersion for administration.

8. The use of claim 1 or method of claim 2, wherein said polymer is provided in the form of a device or a coating thereof for administration.

9. The use or method of claim 8, wherein said device is selected from the group consisting of sutures, stents, vascular grafts, stent-graft combinations, meshes, tissue scaffolds, pins, clips, staples, films, sheets, foams, anchors, screws and plates.

10. The use of claim 1 or method of claim 2, further comprising administering a bioactive agent simultaneously with or separately from said polymer.

11. The use or method of claim 10, wherein said bioactive agent is an anti-inflammatory compound selected from the group consisting of a COX-2 inhibitor, a tetracycline compound, a non-steroidal anti-inflammatory drug (NSAID), a corticosteroid, a matrix metalloprotease inhibitor, a local anaesthetic, glucosamine, chondroitin sulfate and collagen hydrolysate.

12. The use or method of claim 10, wherein said bioactive agent comprises cells, and said polymer is processed into a device or a coating of a device for delivering said cells into a site within said subject.

13. The use or method of claim 12, wherein said cells are capable of producing insulin at said site within said subject.

14. The use of claim 1 or method of claim 2, wherein said monoglyceride is selected from the group consisting of monostearoyl glycerol, monopalmitoyl glycerol, monomyrisitoyl glycerol, monocaproyl glycerol, monodecanoyl glycerol, monolauroyl glycerol, monolinoleoyl glycerol, monooleoyl glycerol, and combinations thereof.

15. The use of claim 1 or method of claim 2, wherein said polybasic acid is selected from the group consisting of succinic acid, glutaric acid, adipic acid, pimelic acid, suberic acid, sebacic acid, diglycolic acid, malic acid, tartaric acid, citric acid, fumaric acid, maleic acid, and a combination thereof.

16. The use of claim 1 or method of claim 2, wherein said derivative of a polybasic acid is selected from an anhydride, an ester, an acid halide, and combinations thereof.

17. The use of claim 1 or method of claim 2, wherein said polyether is a water-soluble linear or branched alkylene oxide, selected from the group consisting of a poly(ethylene oxide), a poly(propylene oxide), a poly(tetra methylene oxide), a polymer comprising a combination of ethylene oxide, propylene oxide or tetramethylene oxide units, and a combination thereof.

18. The use of claim 1 or method of claim 2, wherein said polymer is a copolymer.

19. The use of claim 1 or method of claim 2, wherein said polymer comprises an end-capping moiety selected from the group consisting of alkyl, alkenyl, alkynyl, acrylates, methacrylates, amines, isocyanates and isothiocyanates.

20. The use of a polymer for the manufacture of a medicament for inhibiting or reducing inflammatory responses during or after cell or tissue implantation in a subject comprising mixing said cell or tissue with the polymer, wherein said polymer is the reaction product of a polybasic acid or a derivative thereof, a monoglyceride, and a polyether.

21. A method of inhibiting or reducing inflammatory responses during or after cell or tissue implantation in a subject, comprising mixing said cell or tissue with a polymer which is the reaction product of a polybasic acid or a derivative thereof, a monoglyceride, and a polyether; and implanting the mixture into said subject.

22. The use or claim 20 or method of claim 21, wherein said cell comprises cells capable of producing insulin upon implantation.

23. The use or claim 20 or method of claim 21, wherein said polymer is manufactured as a scaffold or a coating of a scaffold suitable for seeding and delivery of said cell or tissue.

24. The use of a polymer for the manufacture of a scaffold made of or coated with the polymer for treating diabetes comprising seeding cells into said scaffold, wherein said cells are capable of producing insulin upon implantation, and wherein said polymer is the reaction product of a polybasic acid or a derivative thereof, a monoglyceride, and a polyether.

25. A scaffold made of or coated with a polymer and comprising cells seeded into said scaffold, wherein said cells are capable of producing insulin upon implantation, and wherein said polymer is the reaction product of a polybasic acid or a derivative thereof, a monoglyceride, and a polyether.

26. A method of treating diabetes in a subject, comprising providing a scaffold made of or coated with a polymer, wherein said polymer is the reaction product of a polybasic acid or a derivative thereof, a monoglyceride, and a polyether; and seeding cells into said scaffold, wherein said cells are capable of producing insulin upon implantation; and implanting said scaffold into said subject.
